# EUROPEAN PATENT APPLICATION

(11) **EP 3 932 535 A1**
(43) Date of publication of application: **05.01.2022**
(21) Application number: 20183476.9
(22) Date of filing: 01.07.2020
(51) Int. Cl.: B01J 13/00, B01D 61/00, B01J 20/02, B01J 20/06, B01J 20/22, B01J 20/24, B01J 20/30

(54) **MANUFACTURING METHOD FOR POLYNUCLEAR IRON COMPOUNDS STABILIZED BY CARBOHYDRATES AND/OR HUMIC ACID**

(71) Applicant: Vifor Fresenius Medical Care Renal Pharma, Ltd., 9001 St. Gallen (CH)
(72) Inventor: Philipp, Erik, 9320 Arbon (CH); Kammerer, Michael, 78464 Konstanz (CH); Müller, Hans-Martin, 9032 Engelburg (CH)
(74) Representative: Gille Hrabal Partnerschaftsgesellschaft mbB Patentanwälte

(57) **Abstract**

The invention relates to a method of preparation of a polynuclear iron compound stabilized by carbohydrates and/or humic acid or forming a complex with carbohydrates and/or humic acid using a pressure-driven filtration process.

## Description

The present invention relates to an improved method of preparation of a polynuclear iron compound stabilized by carbohydrates and/or humic acid or forming a complex with carbohydrates and/or humic acid.

A number of methods for synthesizing iron compounds stabilized by or complexed with carbohydrates are well known and have been described in the prior art.

EP 0 868 125 discloses a process of producing a polynuclear iron oxihydroxide stabilized by carbohydrates and/or humic acid, in which after precipitation of beta-iron oxihydroxide said compound is washed with water several times by decanting, filtering or centrifuging in order to remove the halide salt by-product of the reaction. To the washed and filtered product a small amount of water is then added to yield a hydrogel, to which carbohydrates and or humic acid can subsequently be added.

Similarly, EP 2 319 804 and WO 2009/150232 describe processes of preparing iron(III)-based phosphate adsorbents comprising the steps of mixing an iron salt with an aqueous base, isolating the resulting precipitate by decantation, filtration or centrifugation, optionally washing the precipitate followed by one of the above isolation steps, re-suspending the precipitate with the minimum amount of water and adding one or more carbohydrates and/or humic acid followed by an isolation step of spray-drying or fluidized bed spray-drying.

All of the processes known from the prior art require considerable effort or involve problems where the steps of washing, filtering and concentrating the suspension obtained after allowing the iron salt to react with the aqueous base are concerned.

Decantation requires manpower and time and cannot be conducted on an industrial scale. Conventional pressure-driven and non-pressure driven filtration and centrifugation methods lead to blocking due to filter cakes or clogging of equipment. These methods require time, give rise to high water consumption and are not energy-efficient due to slower removal of liquids through the build-up of filter cakes, in particular when several washings of said filter cakes are required. Centrifugation requires considerable cleaning efforts and thus delays the process, in particular when carried out on a large scale. Additional problems associated with all of the above filtration methods are the low weight percentage of iron obtained in the material separated by filtration, the iron loss and the insufficient removal of reaction by-products.

Thus, there was a need to develop an improved process for the manufacture of polynuclear iron compounds stabilized by or complexed with carbohydrates and/or humic acid. Said improved process was to comprise more efficient washing, filtration and concentration steps thereby providing an optimized permeate flux, minimal iron loss, a suspension concentration suitable for drying, thereby providing an end- product with a suitable sodium content of ≤ 0.8 % m/m and with higher purity than the products obtainable by the conventional methods. Further, said process had to facilitate easier cleaning and sanitization procedures, as well as being suitable for manufacturing on an industrial scale.

### Summary

The present invention provides a method of preparing a polynuclear iron compound stabilized by carbohydrates and/or humic acid or a polynuclear iron compound forming a complex with carbohydrates and/or humic acid, said method comprising the steps of
a) mixing an aqueous basic solution with a solution of an iron salt comprising halide ions to form a suspension,
b) leaving said suspension to stand, optionally with occasional stirring,
c) washing the suspension and removing the halide salt by-product comprised in the permeate by means of pressure-driven filtration across at least one membrane,
d) concentrating the suspension to obtain a hydrogel,
e) adding one or more carbohydrates and/or humic acid to the hydrogel and, thereby forming the polynuclear iron compound stabilized by carbohydrates and/or humid acid or the polynuclear iron compound forming a complex with carbohydrates and/or humic acid and
f) drying the product obtained in step e) and
g) optionally, granulating the dried product obtained in step f),
wherein said method is characterized in that steps c) and d) are carried out simultaneously with continuous agitation of the suspension.

### Detailed Description of the Invention

It has been surprisingly found that an improved method of preparation of a polynuclear iron compound stabilized by carbohydrates and/or humic acid or a polynuclear iron compound forming a complex with carbohydrates and/or humic acid overcoming the disadvantages of the prior art can be provided in the form of a method comprising the following steps:
a) mixing an aqueous basic solution with a solution of an iron salt comprising halide ions to form a suspension,
b) leaving said suspension to stand, optionally with occasional stirring,
c) washing the suspension and removing the halide salt formed as a reaction by-product comprised in the permeate by means of pressure-driven filtration across at least one membrane,
d) concentrating the suspension to obtain a hydrogel,
e) adding one or more carbohydrates and/or humic acid to the hydrogel, thereby forming the polynuclear iron compound stabilized by carbohydrates and/or humid acidor the polynuclear iron compound forming a complex with carbohydrates and/or humic acid and
f) drying the product obtained in step e) and
g) optionally, granulating the dried product obtained in step f),
wherein said method is characterized in that steps c) and d) are carried out simultaneously with continuous agitation of the suspension.

The iron salt used in step a) is preferably a water-soluble iron salt. More preferably, it is a water-soluble iron(III) salt. Iron(III) salts of organic or inorganic acids can be used, for instance iron(III) chloride, iron(III) bromide, iron(III) sulfate and/or iron(III) nitrate. Preferably, iron(III) chloride is used.

The basic solution used can be an alkaline or an earth metal alkaline solution. In particular, it is an alkali carbonate, an alkali bicarbonate or an alkali hydroxide solution e.g. sodium hydroxide or potassium hydroxide solution. Preferably, an akali carbonate or an alkali bicarbonate solution is used. Particularly preferred is a sodium carbonate or a sodium bicarbonate solution.

The pH of the resultant suspension of iron salt in basic solution is greater than 3, preferably between 5 and 10, more preferably between 6 and 8 and even more preferably between 6.5 and 7.5.

The carbohydrates to be added to the hydrogel in step e) can be water-soluble or water-insoluble and are chosen from the group of agarose, dextran, dextran derivatives, dextrin, maltodextrin, cellulose, cellulose derivatives, maltose, lactose, sucrose, alditols such as mannitol, sorbitol or xylitol, corn starch, wheat starch, rice starch, maize starch, pea starch, potato starch, pre-gelatinized starch and/ or a mixture thereof. Preferably, the carbohydrate to be added to the hydrogel in step e) is sucrose , potato starch or pregelatinized starch or a mixture of any two or any three of these carbohydrates.

One or more carbohydrates and/or humic acid are added in step e) to the hydrogel obtained in step d) in such amounts that the iron content of the dried and optionally granulated polynuclear iron compound obtained in steps f) or g), respectively, which is either stabilized or complexed by carbohydrates and/or humic acid, comprises a maximum of 50 wt% iron. Preferably, the dried and optionally granulated end-product comprises between 10 to 50 wt% of iron. More preferably, the wt% of iron is between 10 to 40 wt%, 10 to 30 wt%, 10 to 20 wt%, 50 to 40 wt%, 50 to 30 wt% or 50 to 20 wt% . More preferably the wt% of iron is 40 wt%, 30 wt%, 20 wt% or 10 wt%. Most preferably, the dried and optionally granulated end-product comprises 20 wt% of iron.

The amount of carbohydrates and/or humic acid added to the hydrogel in step e) is based on the iron content of said hydrogel obtained in step d). In a preferred embodiment, two or three different carbohydrates are added to the hydrogel obtained in step d). In a particularly preferred embodiment, three carbohydrates are added to the hydrogel obtained in step d). In a particularly preferred embodiment, these three carbohydrates are sucrose, potato starch and pre-gelatinized starch.

In a prefrerred embodiment two or three different carbohydrates are added to a hydrogel of iron(III) oxihydroxide. In a yet more preferred embodiment, sucrose, potato starch and pre-gelatinized starch are added to a hydrogel of iron(III) oxihydroxide.

Carbohydrate(s) are added to the hydrogel obtained from step d) in an overall ratio of Fe : carbohydrate(s) of between 1:1 and 1:5, more preferably in a ratio orf 1:1 to 1:4, 1:1 to 1:3 or 1:1 to 1:2. Particularly preferred is a ratio of Fe to carbohydrate(s) of 1:3.

If two different carbohydrates are added to the hydrogel obtained from step d), the ratio of Fe : Carbohydrate 1) : Carbohydrate 2) can be 1.0 : 1.0 : 1.0, 1.0 : 0.5 : 1.5, 1.0 : 1.5 : 1.5, 1.0 : 1.0 : 2.0, 1.0 : 0.5 : 2.5, 1.0 : 2.0 : 2.0, 1.0 : 1.5 : 2.5, 1.0 : 1.0 : 3.0, 1.0 : 0.5 : 3.5, 1.0 : 4.5 : 0.5, 1.0 : 4.0 : 1.0, 1.0 : 3.5 : 1.5, 1.0 : 3.0 : 2.0 or 1.0 : 2.5 : 2.5, wherein Carbohydrate 1) and Carbohydrate 2) are any of the carbohydrates listed above.

If three different carbohydrates are added to the hydrogel obtained from step d), the ratio Fe : Carbohydrate 1) : Carbohydrate 2) : Carbohydrate 3) can be 1.0 : 0.5 : 0.5 : 1.0, 1.0 : 1.0 : 1.0 : 1.0, 1.0 : 1.5 : 0.5 : 1.0, 1.0 : 2.0 : 0.5 : 0.5, 1.0 : 3.0 : 0.5 : 0.5, 1.0 : 2.5 : 1.0 : 0.5, 1.0 : 2.0 : 1.0 : 1.0, 1.0 : 2.0 : 1.5 : 0.5, 1.0 : 1.5 : 1.5 : 1.0, 1.0 : 4.0 : 0.5 : 0.5, 1.0 : 3.5 : 0.5 : 1.0, 1.0 : 3.0 : 1.5 : 0.5, 1.0 : 3.0 : 1.0 : 1.0, 1.0 : 2.5 : 0.5 : 2.0, 1.0 : 2.5 : 1.0 : 1.5, 1.0 : 2.0 : 0.5 : 2.5, 1.0 : 2.0 : 1.0 : 2.0 or 1.0 : 2.0 : 1.5 : 1.5, wherein Carbohydrate 1), Carbohydrate 2) and Carbohydrate 3) are any of the carbohydrates listed above.

In a particularly preferred embodiment, sucrose, potato starch and pre-gelatinized starch are added to a hydrogel of iron(III) oxihydroxide obtained from step d) in a ratio of Fe : sucrose : potato starch : pre-gelatinized starch of 1.0 : 1.5 : 1.0 : 0.5.

The suspension of an iron salt and an aqueous basic solution obtained from step a) is left to stand in step b) for at least 0.5 hours. Preferably, the suspension of an iron salt and an aqueous basic solution obtained from step a) is left to stand in step b) between 0.5 and 5.0 hours. Preferably, it is left to stand between 0.5 and 4.5 hours, 0.5 and 4.0 hours, 0.5 and 3.5 hours, 0.5 and 3.0 hours, 0.5 and 2.5 hours, 0.5 and 2.0 hours, 0.5 and 1.5 hours, 0.5 and 1.0 hours. Optionally, the suspension can be stirred continuously or occasionally during this time, for instance, for periods of 10, 20, 30, 40, 50 and 60 minutes, for example at a time with interruptions of about 10 minutes between stirrings.

From steps a) and b) a polynuclear iron compound is obtained as a precipitate, which is still in suspension. Said polynuclear iron compound is preferably a polynuclear iron(III) compound. In said suspension is also comprised the halide salt formed as a reaction by-product. Preferably, said halide salt is sodium chloride.

In order to desalinate and purify the polnuclear iron compound the by-product is removed by washing the suspension with deionized water in process step c). The washing is accompanied by pressure-driven filtration across at least one membrane such that the polynuclear iron compound is retained and the by-product comprised and dissolved in the permeate is removed. Washings are repeated until the permeate has a conductivity of ≤ 2 mS/cm.

At the same time, this filtration method leads to a concentration of the retained polynuclear iron compound to a hydrogel by the removal of water in step d).

The washing, and concentrating steps c) and d), respectively, are further carried out simultaneously with continuous agitation. This has the effect that the halide salt by-product is removed from the hydrogel by water, transported across at least one membrane against a concentration gradient by applying a pressure, while the formation of a filter cake on the at least one membrane, which would slow down the filtration process, is prevented.

Surprisingly, it has been found that a process according to the present invention utilizing pressure-driven filtration and concentration while simultaneously agitating the suspension to be filtered and concentrated in a continuous fashion is capable of providing a convenient way of combining both process steps in a time and energy-efficient manner. Thus the desired hydrogel product is obtained at a suitable concentration level for the ensuing process step e) of carbohydrate and/or humic acid addition with minimal iron loss. It has further been found that cleaning and sanitization procedures do not have to be carried out as frequently as the filtration apparatus used is less prone to blocking from filter-cake-build-up.

A suitable hydrogel, i.e. a concentrated desalinated suspension in water of a polynuclear iron(III) compound has an elemental iron content of approximately 10 % (m/m) and an elemental iron content of between 5 to 9 % (m/m), preferably 7 to 8 % (m/m) based on the weight of the suspension and a sodium chloride concentration of approximately 0.1 %.

The continuous agitation applied to the washing and concentrating steps c) and d) can be effected by accelerating the solution with moving objects introduced into the suspension such as stirrers but can also be brought about by rotary acceleration such as shaking , vibrating or other movements of the filtration apparatus as such containing the suspension. Advantageously, the continuous agitation of the suspension prevents filter cake build-up on filtration membranes thereby preventing obstruction and rendering the entire process time and energy efficient.

The at least one membrane used has pore sizes of between 50 to 200 nm. Below this range, filtration is possible but time-consuming and occurs only at greatly reduced volumes. Above this range, loss of the synthesized iron(III) compound would occur. Suitable membranes are stable over a pH range of 2 to 11, preferably over a range of 1 to 14.

Typical membrane surfaces are organic membranes surfaces such as polyethersulfone or polyvinylidene fluoride (PVDF) or inorganic membrane surfaces such as ceramics. Preferably, a rotating membrane is used. Even more preferably, the at least one membrane is disposed about a rotating shaft in a multishaft disk (MSD) filtration system.

Further, an additional concentration step may precede washing step c). Said concentration is carried out by a factor of at least 1.5 of the suspension volume. Preferably it is conducted by a factor of 1.5 to 2 of the suspension volume, even more preferably by a factor of 1.5 to 1.8 of the suspension volume. Carrying out such a concentration of the suspension before the hydrogel is washed has the advantageous effect that less water is consumed and less waste water is produced overall during the purification and concentration steps of the procedure. However, the membrane operates under extreme conditions leading to increased wear.

Alternatively, it is also possible to only wash the original volume of the suspension obtained until the required conductivity of the permeate has been reached and to carry out the concentration by the same factors of the original volume as mentioned above. The advantage of this process is that the membrane will be exposed to less stress making it more durable and a larger volume can be filtered during any given time unit. However, water consumption is high with large volumes of waste water.

Further, process steps a) to e) are followed by a drying step f), wherein the product obtained from step e) is dried to a loss on drying (LOD) value of ≤ 10% (m/m). Typical drying methods are spray-drying and vacuum-belt drying . The dry end-product has a particle size distribution, wherein at least 90 % of the particles have a particle size within the range of 4 to 200 µm and d50 is in the range of between 40 µm to 100 µm and a sodium content of ≤0.8 % (m/m), preferably ≤ 0.5 % (m/m).

Optionally, drying step f) is followed by a granulation step g) using conventional granulation methods.

The present invention further relates to the polynuclear iron compound stabilized by carbohydrates and/or humic acid or the polynuclear iron compound forming a complex with carbohydrates and/or humic acid, obtainable by the new method described herein.

The present invention further relates to a polynuclear iron compound stabilized by carbohydrates and/or humic acid or a polynuclear iron compound forming a complex with carbohydrates and/or humic acid having a higher purity, i.e. a lower amount of by-products, than the products obtainable by the conventional methods.

The present invention further relates to the polynuclear iron compound hydrogel obtainable in the process steps a) to d) as described herein.

The present invention further relates to a polynuclear iron compound hydrogel, wherein the polynuclear iron (III) compound is present in the form of a concentrated suspension in water and being characterized by a defined content of elemental iron of approximately 10 % (m/m) or of between 5 to 9 % (m/m), preferably 7 to 8 % (m/m) based on the weight of the suspension. The hydrogel may further be characterized by a low sodium chloride concentration of ≤ 0.5 % (m/m), preferably of approximately 0.1 % (m/m).

The polynuclear iron compound stabilized by carbohydrates and/or humic acid or the polynuclear iron compound forming a complex with carbohydrates and/or humic acid, as well as the polynuclear iron compound hydrogel according to the present invention are in particular characterized by a reduced amount of undesired by-products selected from Na, CO₃, SO₄ and CI and mixtures thereof.

Conventional methods usually result in polynuclear iron products having a Na-content of approximately 0.8 % (m/m). Surprisingly, the new process allows to reduce the Na-content in the resulting polynuclear iron compound to below 0.8 % (m/m), in particular below 0.7 % (m/m), below 0.6 % (m/m) and preferably ≤ 0.5 % (m/m). Accordingly, an aspect of the present invention relates to a polynuclear iron compound stabilized by carbohydrates and/or humic acid or a polynuclear iron compound forming a complex with carbohydrates and/or humic acid, as well as a polynuclear iron compound hydrogel according to the present invention having a Na-content of ≤ 0.5 % (m/m), ≤ 0.4 % (m/m), ≤ 0.3 % (m/m), or ≤ 0.2 % (m/m). The polynuclear iron compound stabilized by carbohydrates and/or humic acid or the polynuclear iron compound forming a complex with carbohydrates and/or humic acid, as well as the polynuclear iron compound hydrogel according to the present invention can be characterized by having a Na-content in a range of 0.2 to ≤ 0.5 % (m/m), or of 0.2 to 0.4 % (m/m), or of 0.2 to 0.3 % (m/m).

Conventional methods further usually result in polynuclear iron products having a CO₃-content of approximately 1.5 % (m/m). Surprisingly, the new process allows to reduce also the CO₃-content in the resulting polynuclear iron compound to ≤ 1.5 % (m/m). A lower CO₃-content is advantageous to reduce the release of gaseous CO₂ during storage, allowing to provide a more stable medical product. Further, a reduced release of CO₂ from the product is advantageous upon administration allowing to provide a medical product with improved digestibility, which is advantageous with respect to patient's compliance and tolerability of the medical product.

Accordingly, an aspect of the present invention relates to a polynuclear iron compound stabilized by carbohydrates and/or humic acid or a polynuclear iron compound forming a complex with carbohydrates and/or humic acid, as well as a polynuclear iron compound hydrogel according to the present invention having a CO₃-content of ≤ 1.5 % (m/m).

Further, the new process as described herein surprisingly provides a polynuclear iron compound stabilized by carbohydrates and/or humic acid or a polynuclear iron compound forming a complex with carbohydrates and/or humic acid (the final dried product) having an average particle size distribution in the range of about 4 to 200 µm, in particular of about 4 to 100 µm, more particularly 4 to 50 µm, very particularly between 7 to 10 µm. Preferably, the polynuclear iron compound stabilized by carbohydrates and/or humic acid or the polynuclear iron compound forming a complex with carbohydrates and/or humic acid (the final dried product) can be characterized by a particle size distribution, wherein at least 40 %, at least 50 %, at least 60 %, at least 70 %, at least 80 % or at least 90 % of the particles have a particle size within the range of about 4 to 200 µm, or between about 4 to 100 µm, or between about 4 to 50 µm, or between about 7 to 10 µm.

The d50 particle size distribution of the dried particles of the polynuclear iron compound stabilized by carbohydrates and/or humic acid or the polynuclear iron compound forming a complex with carbohydrates and/or humic acid (related to the volume of the particles) is preferably in a range of between 40 µm to 100 µm.

The term "d50 particle size distribution" means that 50% (per volume) of the particles have a particle size above or below the defined d50 value expressed in µm. Particle size distributions might be measured using Sieve analysis, or laser diffraction (international standard ISO 13320-1), or electronic sensing zone, light obstruction, sedimentation or microscopy which are procedures well known by the person skilled in the art. Sieving is one of the oldest methods of classifying powders by particle size distribution. A further method includes the determination of the volume particle size distribution by TEM (see e.g. Clariant Analytical Services TECHNICAL SHEET 106 TEM-Partikelgröße). Such methods are well known and described in the art such as in any analytical chemistry text book or by the United State Pharmacopeia's (USP) publication USP-NF (2004-Chapter 786-(The United States Pharmacopeial Convention, Inc., Rockville, Md.)) which describes the US Food and Drug Administration (FDA) enforceable standards. The used techniques are e.g. described in Pharmaceutical dosage forms: volume 2, 2nd edition, Ed.: H. A. Lieberman, L. Lachman, J. B. Schwartz as a good example. It also mentions (page 187) additional methods: Electronic sensing zone, light obstruction, air permeation, sedimentation in gas or liquid. However, the values of the particle size distributions used in the present invention are generally obtained by the Laser diffraction analytical technologies (see for example http://pharmazie-lehrbuch.de/kapitel/3-1.pdf). More specifically the particle size distributions are obtained according to the invention with a LS 13 320 Laser Diffraction Particle Size Analyzer of Beckmann Coulter thereby relying in particular on the corresponding "LS 13 320 Laser Diffraction Particle Size Analyzer Instructions For Use PN B05577AB (October 2011)" using in particular the complete Mie theory. These laser diffraction analytical technologies yield volume weighted distributions. Here the contribution of each particle in the distribution relates to the volume of that particle (equivalent to mass if the density is uniform), i.e. the relative contribution will be proportional to size. More specifically the particle size distribution (PSD) in accordance with the present invention is carried out with a 20 g sample of the dried polynuclear iron compound stabilized by carbohydrates and/or humic acid or dried polynuclear iron compound forming a complex with carbohydrates and/or humic acid (i.e. with the final dried product) which is analyzed with a laser particle size analyzer Beckman Coulter LS 13320 equipped with a dry powder system. A run length of approx 25 seconds and an obscuration of 6 - 10% is applied. The PSD is calculated from the cumulative percentage undersize size distribution using a computer program. Further details are shown in the Example below.

### Examples

A suspension of polynuclear iron (III) oxihydroxide was prepared according to the method described in EP 0 868 125 B1.

A number of pressure-driven and non-pressure-driven methods for the desalination and filtration of suspensions comprising polynuclear iron (III)oxihydroxide at industrial scale were investigated. In this context, industrial scale refers to production batches of the dried product obtained from manufacturing step f)of between 0.2 to 2 tons.

### Non pressure-driven filtration using filter plates

The suspension comprising polynuclear iron (III) oxihydroxide was filtered using ordinary non-pressure-driven filtration with filter plates. The filter cake was washed several times followed by concentration of the solution.

This method was found to be suitable at laboratory scale only. At manufacturing scale, water and energy consumption was very high due to the filter-cake-build-up. The process was very slow and the resultant hydrogel did not have the required iron content.

### Non pressure-driven filtration using a flood-filtration system

A flood filtration system (MAVADISC^{®}, MAVAG Inc.) operating in closed cycle and comprising a filter unit of several filtration plates arranged horizontally one above the other with each filtration plate being mounted on a rotating shaft. Filtration and washing of a filtered product occurs without an externally applied pressure and while the filtration plates ae rotated about the rotating shaft.

During feasability trials it was found that filtration of the hydrogel obtained from manufacturing step e) was possible but inefficient due to the fact that no pressure was applied. A larger filter area would have been needed, which would have been difficult to operate.

### Pressure-driven filtration using tubular membranes

A polypropylene tubular microfiltration membrane module system was used. Such a filtration system works by processing the incoming feed suspension with an applied pressure across several tubular membranes, instead of into a filter. During filtration, any material smaller than the membrane pores of 200 nm , i.e. the by-product, passes through the membranes against a concentration gradient while suspended particulates remain in the retentate stream.

The tubular membranes used had diameters of 5.5 mm and a total surface area of 0.025 m². The pressure applied was 1.5 bar and the flow rate of the suspension to be filtered was between 4.0 and 5.5 m/s.

A first experiment at laboratory scale using a 10 kg batch of suspension, which was first concentrated to 5 kg and then washed four times with deionized water yielded the desired hydrogel but resulted in tubular membranes that were blocked by the hydrogel product.

Based on a batch size of 6200L that is to be washed, filtered and concentrated within four hours, a membrane area of 31 m² would be needed. The expected output is 125 L/m²/h. 12400 kg of deionized water would be needed. 3875 kg of permeate would pass through the membrabes per hour giving rise to a total of 15500 kg.

A second experiment with a batch of equal volume, which was carried out after tubular membrane cleaning and regeneration, had to be discontinued due to poor membrane performance. The membrane surface had been modified by the previous filtration step and/or cleaning measures leading to the membrane module system becoming unusable.

While desalination and concentration of a first batch of hydrogel was achieved using this filtration system, the system was unsuitable for repeated procedures.

### Pressure-driven filtration using a multishaft disk (MSD) filtration system

This filtration system available from Westfalia Separation Filtration GmBH , comprises a series of ceramic ultrafiltration disks stacked on a central rotating shaft. Rotation of the filtration disks prevents substantial filter cake build-up while the filtration is driven by the application of pressure to separate the permeate comprising the by-products and the retentate, i.e.polynuclear iron (III) oxihydroxide.

Initially, two laboratory-scale test were conducted using the MSD filtration system to filter polynuclear Fe(III) oxihydroxide. Both tests were conducted on suspension batches of 12 to 16 L. using ceramic membranes of a pore size of 50 nm and a surface area of 0-13 m².

The first batch was processed such that the MSD filtration system concentrated the suspension by a factor of 2. This was followed up with six washings with deionized water of the same volume as the concentrated suspension in order to desalinate the suspension.

The second batch was introduced into the MSD filtration system and first washed six times with volumes of deionized water that were equivalent to the suspension volume before concentrating the suspension during filtration by a factor of 2.

Both tests resulted in a hydrogel having an Fe(III) oxhihydroxide content of > 11% (m/m) and a reduced NaCl content of approximately 0.1%. The fist test involving concentrating the hydrogel before washing it, proved to be more energy-efficient, less water-consuming but was also found to put a greater strain on the ceramic membrane.

Based on these results, two production campaigns were performed under GMP conditions to produce material for clinical trials (4.22 m² membrane area, 200 nm pore size, production of 30 batches with 1000 I suspension each). To minimize the risk of technical problems during these campaigns, the suspension was washed at least 6 times and then concentrated by a factor of 1.5 - 1.8.

The hydrogel obtained had an Fe(III) oxihydroxide content of approximately 10 % (m/m) and a NaCl content of less than 0.1 % (m/m).

### Further Synthesis Steps and the Finished End-Product

After an Fe(III) oxihydroxide hydrogel having an Fe content of between 5 to 9 % (m/m) had been obtained following desalination and concentration of the original suspension, sucrose, potato starch and pre-gelatinized starch were added to the hydrogel with stirring in a mass ratio of iron : sucrose : potato starch : pre-gelatinized starch of 1: 1.5 : 1 : 0.5

It was found that polynuclear iron(III) oxihydroxide stabilized by sucrose, potato starch and pre-gelatinzed starch could be isolated by spray-drying or vacuum-belt drying from the aqueous suspension so that the final product had a loss on drying (LOD) of ≤ 10% w/w, a particle size distribution, wherein at least 40 % of the particles have a particle size within the range of 4 to 200 µm and d50 is in the range of between 40 µm to 100 µm and an NaCl content of ≤0.8 % (m/m).

The settings from laboratory scale could be transferred to production scale with only marginal changes.

Particle Size Distribution measured by laser diffraction

The particle size distribution of the dried polynuclear iron compound stabilized by carbohydrates and/or humic acid or the dried polynuclear iron compound forming a complex with carbohydrates and/or humic acid (i.e. the final dried product) according to the invention is determined as follows. This method and the values described in the present Example are the basis supporting the values included in the present claims and specification.

### Equipment:

Measuring device: e.g. LS 13 320 Laser Diffraction Particle Size Analyzer of Beckmann Coulter, Beckman Coulter International S.A. Switzerland

Sample module: Vacuum pressure dispersion system, e.g. Dry Powder System (Tornado), Beckman Coulter International S.A. Switzerland

### Conditions:

Average vacuum: approx. 24" H₂O; Obscuration 6-10%; Run length approx. 25 seconds.

The Fraunhofer Model is used for data evaluation.

### Procedure:

Introduce 20 g of the sample into the Dry Powder dispersion System.

Measurement: Apply the specified vacuum to transfer the sample and determine the cumulative volume distribution using a laser light diffraction instrument in accordance with the instruction manual. The parameters may be adjusted so that the test dispersion is representative, homogeneous and well dispersed.

Evaluation/assessment: Determine the particle sizes at the undersize values of 50% (d50), and additional values in question, from the cumulative volume distribution.

## Claims

1. Method of preparing a polynuclear iron compound stabilized by carbohydrates and/or humic acid or a polynuclear iron compound forming a complex with carbohydrates and/or humic acid, said method comprising the steps of
a) mixing an aqueous basic solution with a solution of an iron(III) salt comprising halide ions to form a suspension,
b) leaving said suspension to stand, optionally with occasional stirring,
c) washing the suspension and removing the halide salt by-product comprised in the permeate obtained in step b) by means of pressure-driven filtration across at least one membrane,
d) concentrating the suspension to obtain a hydrogel and
e) adding one or more carbohydrates and/or humic acid to the hydrogel obtained in step d),
f) drying of the product obtained in step e) and
g) optionally, granulating the dried product of step f),
**characterized in that** steps c) and d) are carried out simultaneously with continuous agitation of the suspension.

2. Method according to claim 1, wherein the agitation is provided by rotary acceleration and/or by accelerating the solution with moving objects introduced into the suspension.

3. Method according to claim 1 or 2, wherein the at least one membrane has a pore size of between 50 and 200 nm.

4. Method according to claim 3, wherein the at least one membrane is stable over a pH range of 1 to 14, preferably over a pH range of 2 to 11.

5. Method according to claim 3, 4 or 5, wherein the at least one membrane is a ceramic membrane, a polyethersulfone membrane or a polyvinylidene fluoride membrane.

6. Method according to claims 3 to 5, wherein the at least one membrane is a rotating membrane.

7. Method according to claims 3 to 6, wherein the at least one membrane is comprised in a multishaft disk filtration system.

8. Method according to any one of the preceding claims, wherein washing step c) is repeated until the permeate has a conductivity of ≤ 2 mS/cm.

9. Method according to any one of the preceding claims, wherein an additional concentration step precedes the washing step c).

10. Method according to anyone of the preceding claims, wherein concentration of the suspension occurs by a factor of at least 1.5 of the suspension volume.

11. The method according to any of the preceding claims, wherein in step f) the drying is carried out by spray-drying or fluidized bed spray-drying.

12. The method according to any of the preceding claims, wherein the basic solution of step a) is a sodium carbonate solution or a sodium bicarbonate solution, and / or wherein the iron salt of step a) is FeCl₃.

13. The method according to any of the preceding claims, wherein the carbohydrates are chosen from the group of agarose, dextran, dextrin, maltodextrin, dextran derivatives, cellulose, cellulose derivatives, maltose, lactose, mannitol, sorbitol, xylitol, sucrose, corn starch, wheat starch, rice starch, maize starch, pea starch, potato starch and/or pre-gelatinized starch or a mixture thereof, preferably the carbohydrates are chosen from the group of sucrose, potato starch and pregelatinized starch and mixtures thereof.

14. The method according to any of the preceding claims, wherein 1.5 g of sucrose, 1.0 g of potato starch and 0.5 g of pregelatinized starch are used per g of iron in step e).

15. A polynuclear iron compound stabilized by carbohydrates and/or humic acid or a polynuclear iron compound forming a complex with carbohydrates and/or humic acid, obtainable by the method according to any one of claims 1 to 14, or a polynuclear iron compound hydrogel obtainable by the method according to any one of claims 1 to 10 or 12.

16. A polynuclear iron compound stabilized by carbohydrates and/or humic acid or a polynuclear iron compound forming a complex with carbohydrates and/or humic acid, being **characterized by** one or more of the following
- having a Na-content of ≤ 0.5 % (m/m);
- having a CO₃-content of ≤ 1.5 % (m/m);
- having a particle size distribution, wherein at least 50 % of the particles have a particle size within the range of 4 to 200 µm, preferably between 4 to 100 µm, or between 4 to 50 µm,
- having a d50 value in the range of between 40 µm to 100 µm.

17. A polynuclear iron compound hydrogel in the form of a concentrated suspension of a polynuclear iron (III) compound in water having a content of elemental iron of approximately 10 % (m/m), preferably between 5 to 9 % (m/m), preferably between 7 to 8 % (m/m), based on the weight of the suspension, and a sodium chloride concentration of approximately 0.1 % (m/m).
